(19)

Europäisches
Patentamt
European
Patent Office
Office européen
des brevets

(11)　**EP 4 427 731 A1**

(12)　# EUROPEAN PATENT APPLICATION

(43) Date of publication:
　　**11.09.2024　Bulletin 2024/37**

(21) Application number: **24159262.5**

(22) Date of filing: **23.02.2024**

(51) International Patent Classification (IPC):
　　***A61K 8/60*** *(2006.01)*　***A61Q 5/06*** *(2006.01)*

(52) Cooperative Patent Classification (CPC):
　　**A61Q 5/06; A61K 8/60; A61K 8/731**

(84) Designated Contracting States:
　　**AL AT BE BG CH CY CZ DE DK EE ES FI FR GB
　　GR HR HU IE IS IT LI LT LU LV MC ME MK MT NL
　　NO PL PT RO RS SE SI SK SM TR**
　　Designated Extension States:
　　**BA**
　　Designated Validation States:
　　**GE KH MA MD TN**

(30) Priority:　**23.02.2023　NL 2034207**

(71) Applicant: **Gobiotics BV**
　　**4631 SL Hoogerheide (NL)**

(72) Inventors:
　　• **Gonry, Patrick**
　　　**4631 SL Hoogerheide (NL)**
　　• **Piotrowska, Anna**
　　　**4631 SL Hoogerheide (NL)**

(74) Representative: **De Clercq & Partners**
　　**Edgard Gevaertdreef 10a**
　　**9830 Sint-Martens-Latem (BE)**

(54)　　**NATURAL HAIR STYLING COMPOSITIONS AND METHODS FOR STYLING HAIR USING SAME**

(57)　　The present application relates to a hair styling composition comprising an effective amount of a fructooligosaccharide, particularly a branched inulin, more particularly a branched inulin with a degree of polymerization ranging from 25 to 40, and, optionally, an effective amount of a pullulan, and/or a hydroxyalkyl or carboxyalkyl derivative of cellulose. The hair styling compositions of the present application particularly combine good hair styling and fixative properties with hair moistening properties.

EP 4 427 731 A1

## Description

### Field of the invention

The present invention is situated in the field of cosmetics. In particular, the present invention relates to hair styling compositions with styling, shaping, fixative and moistening or moisture retaining properties.

### Background of the invention

Hair styling products make up a significant part of the cosmetic market. They typically contain film forming polymers of synthetic origin as hair styling or hair fixative agent. Conventional synthetic film forming polymers are based on petrochemical polymers and are not biodegradable. In addition, they are considered as micro-plastics, thus contributing to environmental pollution.

In line with current trends, there is much interest in the cosmetic field to develop hair styling compositions based on natural products. However, the synthetic polymers are difficult to replace by natural ingredients without negatively affecting the quality and functionality of the composition. Consumers expect hair products to provide good and durable styling and cosmetic benefits to the hair. For instance, one of the challenges for creating hair styling products, in particular products for styling curls, is providing a product which has strong holding and fixative properties, but keeps the hair or curl soft and does not form a hard cast.

There is thus a need in the art for new hair styling compositions based on natural and biodegradable ingredients, with good hair styling and cosmetic properties.

### Summary of the invention

The inventors have developed a cosmetic preparation for styling hair based on natural and biodegradable ingredients. Compositions comprising a fructooligosaccharide, in particular a branched or mixed inulin, such as a graminan or an agavin, and/or pullulan, and, optionally a cellulose derivative, such as cellulose gum, exhibited styling properties similar to or even exceeding those of hair styling composition comprising synthetic film-forming polymers. In particular, these compositions had excellent hair styling properties, including but not limited to improving and retaining hair curls, with fixative properties that are highly resistant against moisture. Advantageously, these compositions had surprisingly long-term hair moisturizing or moisture retaining properties. In addition, these compositions are easy to apply and provide a natural and lightweight feel and smoothness to the hair.

The present invention generally relates to methods, uses and compositions for hair styling applications, based on a fructooligosaccharide and/or pullulan, and, optionally a hydroxyalkyl or carboxyalkyl derivative of cellulose, particularly based on a branched fructooligosaccharide, and, optionally pullulan and/or a hydroxyalkyl or carboxyalkyl derivative of cellulose.

A first aspect of the present invention provides a hair styling composition, particularly a leave-on hair styling composition, comprising

- a branched fructooligosaccharide having a degree of polymerization between 3 and 100 or between 5 and 100,
- a cosmetically acceptable carrier, and
- optionally, pullulan, a hydroxyalkyl or carboxyalkyl derivative of cellulose or a combination of pullulan and a hydroxyalkyl or carboxyalkyl derivative of cellulose,

characterized in that

the branched fructooligosaccharide is present in a concentration ranging from 0.1 to 25 wt%, particularly from 0.1 to 15 wt%; and
at least one of the branched fructooligosaccharide, the pullulan, if present, and the hydroxyalkyl or carboxyalkyl derivative of cellulose, if present, is present in a concentration of at least 1.5 wt%, with wt% based on the total weight of the composition.

In particular embodiments, the pullulan is present in a concentration ranging from 0 to 25 wt%, particularly from 0 to 15 wt%, the branched fructooligosaccharide and pullulan are present in a combined concentration ranging from 4 to 25 wt%, particularly ranging from 5 to 15 wt%, with wt% based on the total weight of the composition; and, optionally, the hydroxyalkyl or carboxyalkyl derivative of cellulose is present in a concentration ranging from 1.5 to 3.0 wt%, with wt% based on the total weight of the composition.

In further particular embodiments, the composition comprises the hydroxyalkyl or carboxyalkyl derivative of cellulose in a concentration ranging from 1.5 to 3.0 wt%, with wt% based on the total weight of the composition; the pullulan is present in a concentration ranging from 0 to 5 wt%, particularly ranging from 0 to 4 wt%, and the branched fructooligosaccharide and pullulan, if present, are present in a combined concentration of less than 5 wt%, particularly less than 4 wt%.

In particular embodiments, the branched fructooligosaccharide is a branched inulin. More in particular, the branched inulin has a degree of polymerization of at least 20, particularly has a degree of polymerization ranging from 25 to 40, more particularly has a degree of polymerization ranging from 25 to 35 or from 25 to 30.

In particular embodiments, the hydroxyalkyl or carboxyalkyl derivative of cellulose is hydroxyethyl cellulose, hydroxypropyl cellulose or carboxymethyl cellulose, more in particular is carboxymethyl cellulose.

In particular embodiments, the cosmetically acceptable carrier is chosen from water, organic solvents, such as ethanol, and mixtures thereof.

In particular embodiments, the hair styling composition as envisaged herein is essentially free of synthetic hair styling or synthetic hair fixative agents and/or of silicones.

In particular embodiments, the hair styling composition as envisaged herein further comprises one or more of a fragrance, a colorant, a surfactant, a thickening agent, a vitamin or provitamin, a wax, butter or an oil, an antioxidant, a moisturizer, a chelating agent, or a preservative.

In particular embodiments, the hair styling composition as envisaged herein is in the form of a spray, a gel, a foam or mousse, a cream, an emulsion or lotion, a liquid, a wax, or a paste.

A second, related aspect of the present invention relates to a method for styling hair, comprising applying the hair styling composition according to the present invention to the hair and styling the hair with a styling device or by hand.

A further, related aspect of the present invention relates to the use of a branched fructooligosaccharide, in particular a branched inulin as specified elsewhere herein, and, optionally pullulan and/or a hydroxyalkyl or carboxyalkyl derivative of cellulose, for simultaneously styling and moistening hair.

More in particular, the branched fructooligosaccharide, in particular a branched inulin as specified elsewhere herein, and, optionally, pullulan, may be used in curl styling and/or curl retention applications, for providing soft curls when applied to the hair, as evaluated by sensory analysis (touch).

More in particular, the combination of the branched fructooligosaccharide, in particular the branched inulin as specified elsewhere herein, a carboxyalkyl derivative of cellulose, in particular carboxymethyl cellulose, and, optionally, pullulan, may be used in curl styling and/or curl retention applications, for providing hard and strong curls when applied to the hair, as evaluated by sensory analysis (touch).

**Detailed description of invention**

Before the present system and method of the invention are described, it is to be understood that this invention is not limited to particular systems and methods or combinations described, since such systems and methods and combinations may, of course, vary. It is also to be understood that the terminology used he-rein is not intended to be limiting, since the scope of the present invention will be limited only by the appended claims.

As used herein, the singular forms "a", "an", and "the" include both singular and plural referents unless the context clearly dictates otherwise.

The terms "comprising", "comprises" and "comprised of' as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of".

The recitation of numerical ranges by endpoints includes all numbers and fractions subsumed within the respective ranges, as well as the recited endpoints.

The term "about" or "approximately" as used herein when referring to a measurable value such as a parameter, an amount, a temporal duration, and the like, is meant to encompass variations of +/-10% or less, preferably +/-5% or less, more preferably +/-1% or less, and still more preferably +/-0.1 % or less of and from the specified value, insofar such variations are appropriate to perform in the disclosed invention. It is to be understood that the value to which the modifier "about" or "approximately" refers is itself also specifically, and preferably, disclosed.

Whereas the terms "one or more" or "at least one", such as one or more or at least one member(s) of a group of members, is clear *per se,* by means of further exemplification, the term encompasses *inter alia* a reference to any one of said members, or to any two or more of said members, such as, *e.g.*, any $\geq 3$, $\geq 4$, $\geq 5$, $\geq 6$ or $\geq 7$ etc. of said members, and up to all said members.

All references cited in the present specification are hereby incorporated by reference in their entirety. In particular, the teachings of all references herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous. Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

In the present description of the invention, reference is made to the accompanying drawings that form a part hereof, and in which are shown by way of illustration only of specific embodiments in which the invention may be practiced. It is to be understood that other embodiments may be utilised and structural or logical changes may be made without departing from the scope of the present invention. The following detailed description, therefore, is not to be taken in a limiting sense, and the scope of the present invention is defined by the appended claims.

In the present application, the percentages are given by weight, unless otherwise stated.

The inventors have found that a composition comprising a fructooligosaccharide, particularly a branched fructooligosaccharide as defined elsewhere herein, and/or pullulan, and, optionally, a cellulose derivative combines excellent hair styling properties with high moistening or moisture retaining properties. The observed properties are similar to or even exceed the hair styling properties of synthetic film-forming and/or synthetic hair styling polymers. The present composition comprising a fructooligosaccharide, particularly a branched fructooligosaccharide and/or pullulan, and, optionally, a cellulose derivative thus provides a natural and biodegradable alternative for hair styling compositions based on synthetic hair styling polymers.

Within this context, the present invention thus generally relates to compositions for hair styling or hair styling and moistening applications, comprising a fructooligosaccharide and/or pullulan, and, optionally further comprising a hydroxyalkyl or carboxyalkyl derivative of cellulose, particularly to compositions comprising a branched fructooligosaccharides, and, optionally, pullulan and/or a hydroxyalkyl or carboxyalkyl derivative of cellulose, as well as to methods and uses relating to these compositions.

As used herein, the term "hair" refers to a material made up of keratin fibers, particularly natural keratin fibers. In preferred embodiments, the hair as envisaged herein is human hair.

The terms "hair styling" and "hair fixing/hair fixation" are used interchangeably herein and generally refer to the act of shaping or fixing hair into a desired style or configuration, for instance a temporary curl, and maintaining a desired hair style or configuration.

Hair styling compositions and products are used for structuring and fixing or setting the hair style. These compositions typically comprise one or more "film-forming" polymers or agents, used interchangeably herein with the term "fixative" or "fixing" polymers or agents. The term film-forming or fixative agents or polymers indicate any agent or polymer which is capable of providing a shape or style to the hair and/or maintaining an already acquired shape or style, when applied to the hair. When present in an effective amount, these film-forming or fixative agents typically cover the hair with a film, which forms a sheath around the hair and/or link multiple hairs together thus ensuring the shaping and fixing of the hair.

In the context of the present application, the hair styling compositions considered herein are particularly "leave-on hair styling compositions". Such compositions typically remain on the hair for extended periods of time, without immediate removal, such as by rinsing, after being applied to the hair.

As used herein, the term "fructooligosaccharide" refers to a compound made up of repetitive fructose moieties, particularly made up of between 2 and 100 fructose moieties, more particularly made up of between 3 and 100 or between 5 and 100 fructose moieties, wherein the fructose moieties are linked by beta-2,1 and/or beta-2,6 linkages. In case a glucose moiety is present, it is often located at the end of the fructose chain

A particularly preferred fructooligosaccharide is a branched fructooligosaccharide or branched inulin, particularly a branched fructooligosaccharide or branched inulin with a degree of polymerization of at least 20.

The term "linear inulin" as used herein refers to an essentially linear fructooligosaccharide chain made up of beta-2,1 linked fructose moieties, typically further comprising a chain-terminating glucose residue.

The term "branched inulin", also referred to as "mixed inulin", as used herein is used interchangeably with the term "branched fructooligosaccharide" or "mixed fructooligosaccharide" and refers to a fructooligosaccharide, particularly made up of between 2 and 100 fructose moieties, i.e. having a degree of polymerisation between 2 and 100, more particularly made up of between 3 and 100 or between 5 and 100 fructose moieties, i.e. having a degree of polymerisation between 3 and 100 or between 5 and 100, wherein the fructose moieties are linked by both beta-2,1 and beta-2,6 linkages. More in particular, the terms "branched inulin", "mixed inulin", "branched fructooligosaccharide" or "mixed fructooligosaccharide" refer to a fructooligosaccharide containing a first set of blocks or short linear chains or segments comprising beta-2,1 linked fructose moieties and further containing a second set of blocks or short linear chains or segments comprising beta-2,6 linked fructose moieties.

Particular examples of branched/mixed inulin include graminan or graminin-type fructoologisaccharide, and agavin. Agavin, or agave inulin, is a branched fructooligosaccharide isolated from agave (*Agave* spp.).

As used herein, the degree of polymerization (DP) refers to the average degree of polymerization. The determination of the linkage type (beta-2,1 or beta-2,6) and the (average) degree of polymerization of a fructooligosaccharide are generally known to the skilled person. In particular, the linkage type and average degree of polymerization are determined as described in Mancilla-Margalli and Lopez (J. Agric. Food Chem, 2006, 54, 7832-7839 (doi: 10.1021/jf060354v), incorporated herein by reference, wherein the glycosyl linkage is first analysed by gas chromatography following conversion of the fructooligosaccharide to the corresponding partially methylated alditol acetate derivative and subsequent hydrolysis and reduction thereof, and wherein the average DP is estimated as the percent of both terminal and internal glucose was considered as the unity and the percent of each remaining moiety was compared to this value.

For all the embodiments considered herein, an even more particularly preferred fructooligosaccharide is a branched inulin with a degree of polymerization of at least 20, such as a graminin-type inulin or graminan, or an agavin with a degree of polymerization of at least 20. In particular embodiments, the branched inulin or branched fructooligosaccharide, particularly the graminan or agavin, has a degree of polymerization ranging from 25 to 40, more particularly a degree of polymerization ranging from 25 to 35 or from 25 to 30. In particularly preferred embodiments, the branched inulin is agave inulin, i.e. a branched fructooligosaccharide isolated from agave (*Agave* spp.). Advantageously, these branched inulins are water-soluble and combine excellent hair moisturizing or moisture retaining properties with excellent hair styling properties, including but not limited to improving and retaining hair curls, with fixative properties that are highly resistant against moisture. In contrast, linear inulins exhibit a lower water solubility, and tend to generate more sticky and syrupy compositions.

As used herein, the term "pullulan" refers to an alpha-glucan polysaccharide polymer made up of repetitive maltotriose units, which are linked by an alpha-1,6 glycosidic bond, with the maltotriose units consisting of three alpha-1,4 linked glucose moieties. Pullulan is typically produced from the fungus *Aureobasidium pullulans.* In certain embodiments, the pullulan as used in the compositions as envisaged herein is an *Aureobasidium pullulans* ferment. In certain embodiments, the pullulan as used in the compositions as envisaged herein is isolated from an *Aureobasidium pullulans* fermentation. In certain embodiments, the pullulan has a molecular weight ranging from 50 to 250 kDa, as determined by gel permeation chromatography.

As used herein, the term "hydroxyalkyl or carboxyalkyl derivative of cellulose" refers to a cellulose polymer substituted with hydroxyalkyl or carboxyalkyl substituents. In particular embodiments, the alkyl moiety of the hydroxyalkyl or carboxyalkyl substituents is a C1-C6-alkyl, containing between 1 and 6 carbon atoms, more in particular a C1-C4-alkyl or a C1-C3 alkyl, containing between 1 and 4 or between 1 and 3 carbon atoms, respectively. A particularly preferred carboxyalkyl derivative of cellulose is carboxymethyl cellulose, also referred herein as cellulose gum, i.e. a cellulose derivative wherein the cellulose backbone is substituted with carboxymethyl groups ($-CH_2-COOH$) via some of the cellulose hydroxyl groups. Examples of a hydroxyalkyl derivative of cellulose include hydroxyethyl cellulose or hydroxypropyl cellulose, i.e. a cellulose derivative wherein the cellulose backbone is substituted with hydroxyethyl ($-CH_2CH_2OH$) or hydroxypropyl ($-CH_2CH(OH)CH_3$) groups, respectively, via some of the cellulose hydroxyl groups.

In certain embodiments, the hydroxyalkyl or carboxyalkyl derivative of cellulose, particularly hydroxyethyl cellulose, hydroxypropyl cellulose or carboxymethyl cellulose, more particularly carboxymethyl cellulose has a molecular weight ranging from 50 - 700 kDa, as determined by gel permeation chromatography, and a degree of substitution ranging from 0.5 to 1.5, such as from 0.65 to 1.45.

A first aspect of the present invention provides a hair styling composition, particularly a leave-on hair styling composition, comprising a fructooligosaccharide, particularly a branched fructooligosaccharide, or a combination of a fructooligosaccharide, particularly a branched fructooligosaccharide, and pullulan and/or a hydroxyalkyl or carboxyalkyl derivative of cellulose, wherein the composition is effective as a hair styling or hair fixative agent to style or fix hair into a desired configuration. More particularly, the inventors have found that these components have important hair-styling effects and can be combined to ensure hair-styling effects when they are used in amounts which are higher than those used in traditional compositions where these components are used in low concentrations as thickening or viscosity modifying agents, typically in concentrations below 1.0 wt%. Advantageously, the compositions of the present invention further provide a moistening or moisture retaining effect to the hair.

Accordingly, in particular embodiments, the hair styling compositions of the invention comprise a branched fructooligosaccharide, and, optionally, pullulan and/or a hydroxyalkyl or carboxyalkyl derivative of cellulose, wherein at least one of the branched fructooligosaccharide, the pullulan, and the hydroxyalkyl or carboxyalkyl derivative of cellulose is present in a concentration of at least 1.5 wt%, based on the total weight of the composition. It has been found that at elevated concentrations these components have important hair-styling properties and will result in a composition which is effective as a hair styling or hair fixative agent to style or fix hair into a desired configuration.

In more particular embodiments, the invention provides a hair styling composition, particularly a leave-on hair styling composition, comprising

- a branched fructooligosaccharide, particularly having a degree of polymerization between 5 and 100, particularly in a concentration ranging from 0.1 to 25 wt%
- a cosmetically acceptable carrier, and
- optionally, pullulan, a hydroxyalkyl or carboxyalkyl derivative of cellulose or a combination of pullulan and a hydroxyalkyl or carboxyalkyl derivative of cellulose,

wherein at least one of the branched fructooligosaccharide, the pullulan, if present, and the hydroxyalkyl or carboxyalkyl derivative of cellulose, if present, is present in a concentration of at least 1.5 wt%, with wt% based on the total weight of the composition, and

wherein the composition is effective as a hair styling or hair fixative agent to style or fix hair into a desired configuration, more in particular wherein the branched fructooligosaccharide, and, optionally the pullulan, and/or the hydroxyalkyl or carboxyalkyl derivative of cellulose, are present in a combined concentration that is effective as a hair styling or hair fixative agent to style or fix hair into a desired configuration.

Also envisaged herein is a hair styling composition, comprising

- an effective amount of a branched fructooligosaccharide, or an effective amount of the combination of a branched fructooligosaccharide and pullulan, and
- optionally an effective amount of a hydroxyalkyl or carboxyalkyl derivative of cellulose.

As used herein, "an effective amount" of the fructooligosaccharides and/or pullulan, and optionally the hydroxyalkyl or carboxyalkyl derivative of cellulose, or a combination thereof, refers to an amount which is effective as a hair styling or hair fixative agent to style or fixate hair into a desired configuration.

Advantageously, hair styling compositions comprising a branched fructooligosaccharide, or a combination of a branched fructooligosaccharide and pullulan provide strong hair fixative properties, which are resistant to humidity, a soft feel of the shaped hair, and good moisturizing/hydrating or moisture retaining properties. The presence or addition of a hydroxyalkyl or carboxyalkyl derivative of cellulose further contributes to the fixative properties and provide a harder feel to the shaped hair.

In a particular embodiment, the hair styling composition comprises a branched fructooligosaccharide, or a combination of a fructooligosaccharide and pullulan, and, optionally, a hydroxyalkyl or carboxyalkyl derivative of cellulose, wherein:

- the branched fructooligosaccharide is present in a concentration ranging from 0.1 to 25 wt%, particularly from 0.1 to 15 wt%;
- the pullulan is present in a concentration ranging from 0 to 25.0 wt%, particularly from 0 to 15.0 wt%,
- the branched fructooligosaccharide and pullulan are present in a combined concentration ranging from 4.0 to 25.0 wt%, particularly ranging from 5.0 to 15.0 wt%, with wt% based on the total weight of the composition; and
- optionally, the hydroxyalkyl or carboxyalkyl derivative of cellulose is present in a concentration ranging from 1.5 to 3.0 wt%, such as ranging from 1.5 to 2.5 wt%, with wt% based on the total weight of the composition.

It has been found that such compositions have important hair-styling properties and provide soft, durable and moisture resistant styling/fixing properties as well as hydration or moisture imparting or retaining properties to hair, such as for providing soft, durable and moisture resistant curl retention.

In a further particular embodiment, the hair styling composition comprises a branched fructooligosaccharide, or a combination of a branched fructooligosaccharide and pullulan, and a hydroxyalkyl or carboxyalkyl derivative of cellulose, wherein:

- the branched fructooligosaccharide is present in a concentration ranging from 0.1 to 5 wt%, particularly from 0.1 to 4 wt%;
- the hydroxyalkyl or carboxyalkyl derivative of cellulose is present in a concentration ranging from 1.5 to 3.0 wt%, such as from 1.5 to 2.5 wt%, with wt% based on the total weight of the composition;
- the pullulan is present in a concentration ranging from 0 to 5.0 wt%, particularly ranging from 0 to 4.0 wt%, with wt% based on the total weight of the composition, and
- the branched fructooligosaccharide and pullulan are present in a combined concentration of less than 5.0 wt%, particularly less than 4.0 wt%, with wt% based on the total weight of the composition.

It has been found that such compositions have important hair-styling properties and provide strong, durable and moisture resistant styling/fixing properties as well as hydration or moisture imparting or retaining properties to hair, such as for providing durable and moisture resistant curl retention.

In particular embodiments, the hair styling composition comprises a branched fructooligosaccharide, or a combination of a fructooligosaccharide and a pullulan and/or a hydroxyalkyl or carboxyalkyl derivative of cellulose, wherein:

- the branched fructooligosaccharide is a branched inulin with degree of polymerization of at least 20, particularly a branched inulin with degree of polymerization between 25 and 40 or between 25 and 35; and/or the hydroxyalkyl or carboxyalkyl derivative of cellulose is carboxymethyl cellulose, hydroxyethyl cellulose or hydroxypropyl cellulose;
- the branched fructooligosaccharide is present in a concentration ranging from 0.1 to 25 wt%, particularly from 0.1 to 15 wt%; and
- the pullulan is present in a concentration ranging from 0 to 25.0 wt%, particularly from 0 to 15.0 wt%,
- the branched fructooligosaccharide and pullulan are present in a combined concentration ranging from 4.0 to 25.0 wt%, particularly ranging from 5.0 to 15.0 wt%, with wt% based on the total weight of the composition; and
- optionally, the hydroxyalkyl or carboxyalkyl derivative of cellulose is present in a concentration ranging from 1.5 to 3.0 wt%, such as ranging from 1.5 to 2.5 wt%, with wt% based on the total weight of the composition.

In further particular embodiments, the hair styling composition comprises a branched fructooligosaccharide, or a combination of a fructooligosaccharide and pullulan, and a hydroxyalkyl or carboxyalkyl derivative of cellulose, wherein:

- the branched fructooligosaccharide is a branched inulin with degree of polymerization of at least 20, particularly a branched inulin with degree of polymerization between 25 and 40 or between 25 and 35; and/or the hydroxyalkyl or carboxyalkyl derivative of cellulose is carboxymethyl cellulose, hydroxyethyl cellulose or hydroxypropyl cellulose; the branched fructooligosaccharide is present in a concentration ranging from 0.1 to 5 wt%, particularly from 0.1 to 4 wt%; and - the hydroxyalkyl or carboxyalkyl derivative of cellulose is present in a concentration ranging from 1.5 to 3.0 wt%, such as ranging from 1.5 to 2.5 wt%, with wt% based on the total weight of the composition;
- the pullulan is present in a concentration ranging from 0.0 to 5.0 wt%, particularly ranging from 0.0 to 4.0 wt%, and
- the fructooligosaccharide and pullulan are present in a combined concentration of less than 5.0 wt%, particularly less than 4.0 wt%.

The hair styling compositions as envisaged herein are cosmetic compositions and typically further comprise a cosmetically acceptable carrier, i.e. a physiologically acceptable medium, which is compatible with or suitable for application to the skin and hair, particularly human skin, including but not limited the skin of the body, face, or scalp, and human hair, i.e. typically without undue toxicity, instability or allergic response.

For all the embodiments envisaged herein, the cosmetically acceptable carrier may be chosen from water, organic solvents and mixtures thereof. More in particular, the hair styling composition as envisaged herein is an aqueous composition, comprising water or a mixture of water and an organic solvent, particularly ethanol, as cosmetically acceptable carrier. Examples of cosmetically acceptable organic solvents include C2-C4 alcohols, such as ethanol or isopropanol; C2-C6 polyols, such as glycerol or propylene glycol; polyol ethers, such as 2-butoxyethanol or propylene glycol monomethyl ether, and mixtures thereof. Preferably, the cosmetic composition comprises at least 30 wt% or at least 50wt% of water, such as between 50 wt% and 98 wt% or between 65 wt% and 95 wt% of water, based on the total weight of the composition. The pH of the hair styling compositions as envisaged herein is preferably from about 4 to about 9, more preferably from about 4.5 to about 7.5. Buffers and other pH adjusting agents can be included to achieve the desired pH.

The hair styling compositions as envisaged herein provide a natural and biodegradable alternative to hair styling compositions comprising synthetic film-forming polymers. The term "synthetic polymer" or "synthetic film-forming polymer" as used herein refers to a polymer, which is purely synthetic, or not of natural origin, for instance, polymers made by radical polymerization of ethylenically unsaturated monomers or by polycondensation. It is understood that the fructooligosaccharide, particularly the branched inulin, pullulan and/or the hydroxyalkyl or carboxyalkyl derivative of cellulose, such as carboxymethyl cellulose, are each a natural film-forming and/or fixative polymer or agent, with the term "natural" indicating an isolated compound or ingredient which remains essentially unchanged from its natural state, or which has undergone some limited processing to improve its stability or efficacy. The term "natural polymer" means a polymer of natural origin, which remains unchanged from its natural state or a polymer of natural origin which has undergone a chemical, enzymic or physical processing which has modified it from its natural stat, but which retains at least 50% of its molecular structure from the original natural source. In particular embodiments, the hair styling compositions as envisaged herein contain the fructooligosaccharide, particularly the branched inulin, the pullulan and/or the hydroxyalkyl or carboxyalkyl derivative of cellulose, such as carboxymethyl cellulose, as the only fixative polymers. In certain embodiments, in case the hair styling compositions as envisaged herein contain other film-forming or fixing polymers, they are preferably present in an amount of less than 2.0 wt%, less than 1.5 wt% or even less than 1.0 wt%, based on the total weight of the composition.

In particular embodiments, the hair styling compositions as envisaged herein is essentially free of synthetic hair styling or synthetic hair fixative agents and/or of silicones.

For all the embodiments envisaged herein, the hair styling composition as envisaged herein may further comprise one or more cosmetically acceptable ingredients or additives, different from the branched fructooligosaccharide, pullulan or hydroxyalkyl or carboxyalkyl derivative of cellulose as envisaged herein, such as one or more of a fragrance, a colorant, a surfactant, a thickening agent or viscosity modifier, a vitamin or provitamin, a wax, butter or an oil, an antioxidant, a moisturizer, a chelating agent or a preservative. Such cosmetically acceptable ingredients or additives are generally known in the art. It is understood that, in general, the skilled person is able to select one or more of these ingredients or additives and their quantities and optimize the formulation of the compositions according to the present invention and the processes for preparing them on the basis of his general technical knowledge and taking into account the envisaged use, the physical form and desired characteristics of the composition. For instance, in embodiments which include a thickening agent or viscosity modifier, it is typically included in the hair styling compositions as envisaged herein at a level of preferably from about 0.005% to about 1.5 wt%, more preferably from about 0.01% to about 1.0 wt%, based on the total weight of the composition. For instance, a hair styling composition according to the present application in the form of a spray may further comprise 50-80 wt% water, 10-40 wt% ethanol. For instance, a hair styling composition according to the present application in the form of a gel may further comprise 0.2-1 wt% carbomer, 1.0-3.0 wt% glycerine, 0.5-1.0 wt% panthenol. Also, a hair styling composition according to the present application in the form of a cream may further comprise 1.0-2.0 wt% PEG-100 stearate; 1.0-2.0 wt% glyceryl stearate, 5.0-10.0 wt% of capric caprylic triglyceride, 2.0-5.0 wt% of glycerine and 2.0-4.0 wt% cetearyl alcohol.

The hair styling composition as envisaged herein may be in any suitable physical form. In certain embodiments, the composition is in the form of a spray, a gel, a foam or mousse, a cream, an emulsion or lotion, a liquid, a wax, or a paste. Particularly preferred are hair styling compositions according to the present application in the form of a gel or wax. Such compositions may be prepared according to the conventional methods used in the field.

In particular embodiments, the hair styling composition comprises a branched fructooligosaccharide, and, optionally, pullulan and/or a hydroxyalkyl or carboxyalkyl derivative of cellulose, and a cosmetically acceptable carrier, wherein:

- the branched fructooligosaccharide is a branched inulin with degree of polymerization of at least 20, particularly a branched inulin with degree of polymerization between 25 and 40 or between 25 and 35; and/or the hydroxyalkyl or carboxyalkyl derivative of cellulose is carboxymethyl cellulose, hydroxyethyl cellulose or hydroxypropyl cellulose; the branched fructooligosaccharide is present in a concentration ranging from 0.1 to 25 wt%, particularly from 0.1 to 15 wt%;
- the pullulan is present in a concentration ranging from 0 to 25.0 wt%, particularly from 0 to 15.0 wt%,
- the branched fructooligosaccharide and pullulan are present in a combined concentration ranging from 4.0 to 25.0 wt%, particularly ranging from 5.0 to 15.0 wt%, with wt% based on the total weight of the composition;
- optionally, the hydroxyalkyl or carboxyalkyl derivative of cellulose is present in a concentration ranging from 1.5 to 3.0 wt%, such as ranging from 1.5 to 2.5 wt%, with wt% based on the total weight of the composition; and
- the hair styling composition is in the form of a spray, a gel, a foam or mousse, a cream, an emulsion or lotion, a liquid, a wax, or a paste.

In further particular embodiments, the hair styling composition comprises a branched fructooligosaccharide, or a combination of a branched fructooligosaccharide and pullulan, and a hydroxyalkyl or carboxyalkyl derivative of cellulose, and a cosmetically acceptable carrier, wherein:

- the branched fructooligosaccharide is a branched inulin with degree of polymerization of at least 20, particularly a branched inulin with degree of polymerization between 25 and 40 or between 25 and 35; and/or the hydroxyalkyl or carboxyalkyl derivative of cellulose is carboxymethyl cellulose, hydroxyethyl cellulose or hydroxypropyl cellulose;
- the hydroxyalkyl or carboxyalkyl derivative of cellulose is present in a concentration ranging from 1.5 to 3.0 wt%, with wt% based on the total weight of the composition; the branched fructooligosaccharide is present in a concentration ranging from 0.1 to 5 wt%, particularly from 0.1 to 4 wt%;

- the pullulan is present in a concentration ranging from 0 to 5.0 wt%, particularly ranging from 0 to 4.0 wt%,
- the fructooligosaccharide and pullulan are present in a combined concentration of less than 5.0 wt%, particularly less than 4.0 wt%; and
- the hair styling composition is in the form of a spray, a gel, a foam or mousse, a cream, an emulsion or lotion, a liquid, a wax, or a paste.

For all the embodiments envisaged herein, the hair styling compositions as envisaged herein may be contained in any packaging that is suitable for storing cosmetic compositions, such as a bottle, tube, jar, spray bottle or aerosol bottle, and which is adapted to the physical form of the compositions envisaged herein. Accordingly, the invention further relates to containers, such as those described above, comprising the composition of the invention. In particular embodiments the container comprises or is accompanied by instructions for applying the composition of the invention on hair. In further particular embodiments the instructions specify that the product need not be rinsed out of the hair.

A related aspect of the present invention relates to a method for styling hair, comprising applying a hair styling composition as envisaged herein to the hair and styling the hair with a styling device or by hand or fingers. Styling devices are known in the art and include but are not limited to brushes (optionally fixed to hair-dryers), hair-styling devices (also referred to as hair-irons, hair curlers or hair dryers), hair rollers or curls etc.

The hair styling composition may be applied to the hair before, during or after configuring the hair into the desired shape. The hair styling composition may be applied to wet, dry or semidry or damp hair. In particular embodiments, the composition is applied to the hair prior to styling. Also envisaged are methods for preparing hair for styling which involve the step of applying the composition to the hair.

In particular embodiments, the compositions according to the present application are applied to the hair in an amount ranging from 0.1 to 10 g per g of (dry) hair, typically up to 20g per scalp.

In particular embodiments of the methods of the invention the composition is not rinsed out of the hair after styling but remains in the hair.

A further related aspect of the present invention relates to the use of a fructooligosaccharide as described elsewhere herein, particularly a branched fructooligosaccharide as described elsewhere herein, and, optionally, pullulan and/or a hydroxyalkyl or carboxyalkyl derivative of cellulose, for hair styling and hair grooming applications, particularly wherein the fructooligosaccharide is a branched inulin with degree of polymerization of at least 20, particularly a branched inulin with degree of polymerization between 25 and 40 or between 25 and 35; and/or the hydroxyalkyl or carboxyalkyl derivative of cellulose is carboxymethyl cellulose, hydroxyethyl cellulose or hydroxypropyl cellulose.

In particular, the branched fructooligosaccharide as described elsewhere herein, and/or pullulan, and, optionally, a hydroxyalkyl or carboxyalkyl derivative of cellulose may be used for providing durable and moisture resistant styling/fixing properties as well as hydration or moisture imparting or retaining properties to hair, such as for providing durable and moisture resistant curl retention.

Particular embodiments provide for the use of a branched fructooligosaccharide as described elsewhere herein, or the combination of a branched fructooligosaccharide and pullulan, and, optionally, a hydroxyalkyl or carboxyalkyl derivative of cellulose in curl styling applications for providing soft curls, as evaluated by sensory analysis (touch).

Further particular embodiments provide for the use of a branched fructooligosaccharide as described elsewhere herein, or the combination of a branched fructooligosaccharide and pullulan, and a hydroxyalkyl or carboxyalkyl derivative of cellulose, in particular carboxymethyl cellulose, in curl styling applications, for providing hard curls, as evaluated by sensory analysis (touch).

The present invention is further illustrated by the following non-limiting examples.

## Examples

### Example 1 - Hair styling effects of compositions comprising branched inulin with degree of polymerization (DP) of 25-30

The effect of hair styling compositions according to the present invention, comprising either 3.3 wt% branched inulin (agavin), with DP between 25 and 30, and 1.7 wt% pullulan, hereinafter referred to as Gosulin Style 5%, or 6.6 wt% branched inulin (agavin), with DP between 25 and 30, and 3.3 wt% pullulan, hereinafter referred to as Gosulin Style 10%, on curl retention, curl hardness, and resistance against humidity, as well as their moisturizing properties were evaluated and compared with comparative hair styling compositions comprising 5% or 10% PVP K30 (BASF), 5% hydrolyzed corn starch (DOW) or 5% Polyurethane-14 (PU-14) and AMP-Acrylates copolymer (Akzo Nobel). The comparative hair styling compositions comprise well-known, synthetic curl styling polymers and a naturally derived polymer.

The composition of the compositions used in this example are shown in Table 1.

Table 1. Composition of the tested hair styling compositions

| Sample | Ingredients | | |
|---|---|---|---|
| | water | Polymer (wt%) | Euxyl PE9010 (wt%) |
| Gosulin Style 5% | qs | 5 | 1 |
| Gosulin Style 10% | qs | 10 | 1 |
| PVP K30 5% | qs | 5 | 1 |
| PVP K30 10% | qs | 10 | 1 |
| PU-14 and AMP-acrylates copolymer 5% | qs | 5 | 1 |
| PU-14 and AMP-acrylates copolymer 10% | qs | 10 | 1 |
| Hydrolyzed corn starch | qs | 5 | 1 |
| Hydrolyzed corn starch (10%) | qs | 10 | 1 |
| Blank | qs | 0 | 1 |
| Untreated | 0 | 0 | 0 |

A/ Hair pretreatment. The different hair styling compositions were tested on Caucasian, bleached hair swatches (40 cm long and 5 cm wide). Prior to the experiment, the hair swatches were washed with a neutral shampoo, rinsed, and treated with a neutral hair conditioner and dried. The conditioner was used for hair detangling and combing before performing the tests. The composition of the neutral shampoo and the hair conditioner are shown in Table 2 and 3, respectively.

Table 2. Composition of the neutral shampoo.

| Ingredient | Description | Amount (%) |
|---|---|---|
| Water | Aqua | qs |
| MARLINAT 242/28 | Sodium Laureth Sulfate | 9 |
| Sodium Chloride | | 0.5 |
| Perlogen® SF 117 | Water, Glycol Distearate, Laureth-4 | 2 |
| SABOSOL PB | Cocamidopropyl Betaine | 5 |
| SABOAMID DEC | Cocamide DEA | 2 |
| Euxyl PE9010 | Phenoxyethanol, Ethylhexylglycerin | 1 |

Table 3. Composition of the neutral conditioner.

| Ingredient | Description | Amount (%) |
|---|---|---|
| Water | Aqua | qs |
| Cellosize | Hydroxyethylcellulose | 1 |
| Dehyquart® A-CA | Cetrimonium Chloride | 4 |
| Euxyl PE9010 | Phenoxyethanol, Ethylhexylglycerin | qs |
| Sabonal C1618 50/50 | Cetearyl alcohol | 4 |

B/ For the evaluation of curl retention, the different compositions (3 g) were applied on the pretreated dry hair swatches (see section A/ above) and curl on foam curlers.

The curls were left to dry in ambient temperature for 1 day. Next, the hair was untangled and the curls were pinned on a vertical board. The length of each curl was measured. The measurement was repeated after 1 day.

The length of the curl was measured from the base, where the hair was attached to the vertical board with tape, to the bottom of the curl (cm).

The curl retention or curl durability was quantified by a curl retention factor, according to equation (eq 1) and the results are presented in Table 4.

$$Curl\ retention = \frac{L - L_t}{L - L_0} * 100\%$$ (eq 1)

With:

L: length of straight hair swatch
$L_0$ - length of hair curl after untangling
$L_t$ - length of hair curl after 24h

Table 4. Curl retention

| Composition | Curl retention % |
|---|---|
| PVP K30 10% | 82% |
| Gosulin Style 10% | 92% |
| Hydrolysed corn starch 10% | 80% |
| PU-14 and AMP-acrylates copolymer 10% | 95% |

The Gosulin Style composition according to the present invention has a high curl retention power, equal to or even outperforming the curl retention of synthetic polymers and hydrolysed corn starch.

C/ For the evaluation of curl hardness/softness, the different compositions (3 g) were applied on the pretreated dry hair swatches (see section A/ above) and curl on foam curlers.

The curls were left to dry in ambient temperature for 1 day. After this the hair was untangled and the hair hardness/softness was evaluated by stroking the hair swatches. Curl hardness was evaluated in the scale between 1 and 5, whereby score 1 was assigned to the softest swatch and 5 to the hardest. The results are presented in Table 5.

Table 5. Curl hardness/softness

| Composition | Curl hardness |
|---|---|
| PVP K30 5% | 2.5 |
| PVP K30 10% | 3.5 |
| Gosulin Style 5% | 3 |
| Gosulin Style 10% | 3 |
| Hydrolysed corn starch 5% | 5 |
| PU-14 and AMP-acrylates copolymer 5% | 2.5 |

The Gosulin Style compositions according to the present invention provides enough elasticity to the hair to create a soft curl, similar to the synthetic polymers.

D/ For the evaluation of the resistance against humidity, the different compositions (3 g) were applied on the pretreated dry hair swatches (see section A/ above) and curl on foam curlers.

The curls were left to dry in ambient temperature for 1 day. After this the hair was untangled and the length of each curl was measured. During the following 3 hours, the curls were sprayed each 10 minutes with water mist and the length was measured after each hour.

The length of the curl was measured from the base, where the hair was attached to, to the bottom of the curl (cm).

The curl retention factor was evaluated according to equation (eq 1), wherein L and $L_0$ are defined as in section B/ above, and wherein $L_t$ is the length of the curl after 1, 2 or 3 hours. The results are shown in Table 6.

Table 6. Moisture resistance of the curls

| Composition | Curl retention, 1 h | Curl retention, 2h | Curl retention, 3h |
|---|---|---|---|
| PVP K30 10% | 86% | 59% | 45% |
| Gosulin Style 10% | 89% | 62% | 49% |
| Hydrolyzed corn starch 10% | 94% | 69% | 63% |

The Gosulin Style compositions according to the present invention perform better in humid conditions than the synthetic polymer PVP K30.

D/ For the evaluation of the moisturizing properties of the compositions, the different compositions (3 g) were applied on the pretreated dry hair swatches (see section A/ above). The moisture was measured with a corneometer (Corneometer CM 825 (Courage&Khazaka GmbH). In order to perform the measurements correctly, the hair was rubbed to break the cast or film formed by the polymer. The results are shown in Table 7.

Table 7. Moisturising properties of the compositions.

| Time (min) | Untreated | blank | PVP K30 5% | Gosulin Style 5% | Hydrolyzed corn starch 5% |
|---|---|---|---|---|---|
| 30 | 2.1 | 31.3 | 103 | 108 | 84.5 |
| 60 | 1.5 | 3.9 | 32.4 | 78 | 67.5 |
| 120 | 0 | 1.8 | 10.4 | 44.9 | 53.8 |
| 180 | 1.2 | 2.1 | 5.4 | 36.7 | 4.6 |
| 480 | 2.3 | 1.2 | 5.9 | 20.5 | 1.5 |

The Gosulin Style compositions according to the present invention have remarkable, longlasting moisturizing properties.

### Example 2 - Hair styling effects of compositions comprising cellulose gum and branched inulin with degree of polymerization (DP) of 25-30

The effect of hair styling compositions according to the present invention, comprising 1.8% cellulose gum and 0.2% branched inulin, with DP between 25 and 30, hereinafter referred to as Gosulin Hold 2% , on curl retention/durability, curl hardness, and resistance against humidity, as well as their moisturizing properties were evaluated and compared with comparative hair styling compositions comprising 5% or 10% PVP K30 (BASF), 5% hydrolyzed corn starch (DOW) or 5% Polyurethane-14 (PU-14) and AMP-Acrylates copolymer (Akzo Nobel). The comparative hair styling compositions comprise well-known, synthetic curl styling polymers and a naturally derived polymer.

The composition of the compositions used in this example are shown in Table 8.

Table 8. Composition of the tested hair styling compositions

| Sample | Ingredients | | |
|---|---|---|---|
| | Water | Polymers (wt%) | Euxyl PE9010 (wt%) |
| Gosulin Hold 2% | Qs | 2 | 1 |
| PVP K30 5% | Qs | 5 | 1 |
| PVP K30 10% | qs | 10 | 1 |
| PU-14 and AMP-acrylates copolymer | qs | 10 | 1 |
| Hydrolyzed corn starch | qs | 5 | 1 |
| Hydrolyzed corn starch | qs | 10 | 1 |
| Blank | qs | 0 | 1 |
| Untreated | 0 | 0 | 0 |

A/ Hair pretreatment. The different hair styling compositions were tested on Caucasian, bleached hair swatches (40 cm long and 5 cm wide). Prior to the experiment, the hair swatches were washed with a neutral shampoo, rinsed, and treated with a neutral hair conditioner and dried. The conditioner was used for hair detangling and combing before performing the tests. The composition of the neutral shampoo and the hair conditioner are shown above, in Table 2 and 3, respectively.

B/ For the evaluation of curl retention, the different compositions (3 g) were applied on the pretreated dry hair swatches (see section A/ above) and curl on foam curlers.

The curls were left to dry in ambient temperature for 1 day. Next, the hair was untangled and the curls were pinned on a vertical board. The length of each curl was measured. The measurement was repeated after 1 day.

The length of the curl was measured from the base, where the hair was attached to the vertical board with tape, to the bottom of the curl (cm).

The curl retention or curl durability was quantified by a curl retention factor, according to equation (eq 1) and the results are presented in Table 9.

Table 9. Curl retention

| Composition | Curl retention % |
|---|---|
| Gosulin Hold 2% | 98% |
| PVP K30 5% | 90% |
| Hydrolysed corn starch 5% | 96% |
| PU-14 and AMP-acrylates copolymer 5% | 95% |

The Gosulin Hold composition according to the present invention has a high curl retention power, outperforming the tested synthetic polymers and hydrolysed corn starch.

C/ For the evaluation of curl hardness/softness, the different compositions (3 g) were applied on the pretreated dry hair swatches (see section A/ above) and curl on foam curlers.

The curls were left to dry in ambient temperature for 1 day. After this the hair was untangled and the hair's hardness/softness was evaluated by stroking the hair swatches. Curl hardness was evaluated on a scale between 1 and 5, whereby score 1 was assigned to the softest swatch and 5 to the hardest. The results are presented in Table 10.

Table 10. Curl hardness/softness

| Composition | Curl hardness |
|---|---|
| Gosulin Hold 2% | 4 |
| PVP K30 5% | 3 |
| Hydrolysed corn starch 5% | 3.5 |
| PU-14 and AMP-acrylates copolymer 5% | 2.5 |

The Gosulin Hold composition provides fairly hard curls.

D/ For the evaluation of the resistance against humidity, the different compositions (3 g) were applied on the pretreated dry hair swatches (see section A/ above) and curl on foam curlers. The curls were left to dry in ambient temperature for 1 day. After this the hair was untangled and the length of each curl was measured. During the following 3 hours, the curls were sprayed each 10 minutes with water mist and the length was measured after each hour.

The length of the curl was measured from the base, where the hair was attached to, to the bottom of the curl (cm).

The curl retention factor was evaluated according to equation (eq 1), wherein L and $L_0$ are defined as in section B/ above, and wherein $L_t$ is the length of the curl after 1, 2 or 3 hours. The results are shown in Table 11.

Table 11. Moisture resistance of the curls

| Composition | Curl retention, 1 h | Curl retention, 2h | Curl retention, 3h |
|---|---|---|---|
| PVP K30 10% | 86% | 59% | 45% |
| Gosulin Hold 2% | 92% | 71% | 58% |
| Hydrolyzed corn starch 10% | 94% | 69% | 63% |

The Gosulin Hold composition according to the present invention performs better in humid conditions than the synthetic polymer PVP K30 and similar to hydrolyzed corn starch.

D/ For the evaluation of the moisturizing properties of the compositions, the different compositions (3 g) were applied on the pretreated dry hair swatches (see section A/ above). The moisture was measured with a corneometer. In order to perform the measurements correctly, the hair was rubbed to break the cast or film formed by the polymer. The results are shown in Table 12.

Table 12. Moisturising properties of the compositions.

| Time (min) | Untreated | blank | PVP K30 5% | Gosulin Hold 2% | Hydrolyzed corn starch 5% |
|---|---|---|---|---|---|
| 30 | 2.1 | 31.3 | 103 | 123.7 | 84.5 |
| 60 | 1.5 | 3.9 | 32.4 | 121 | 67.5 |
| 120 | 0 | 1.8 | 10.4 | 75.8 | 53.8 |
| 180 | 1.2 | 2.1 | 5.4 | 61 | 4.6 |
| 480 | 2.3 | 1.2 | 5.9 | 11.8 | 1.5 |

The Gosulin Hold composition according to the present invention has remarkable moisture retaining properties.

The Gosulin Hold composition is a composition comprising natural polymers which performs similar to synthetic hair styling polymers, particularly in curl styling applications. It provides a strong hold and a high resistance to humidity and has good moisture retaining properties.

### Example 3 - Stiffness styling effects

The effect of hair styling compositions according to the present invention, in particular the Gosulin Style 5% (as in Example 1) and Gosulin Hold 2% (as in Example 2), on stiffness hold and durability were evaluated and compared with comparative hair styling compositions comprising 5% PVP K30 (BASF), or 5% Polyurethane-14 (PU-14) and AMP-Acrylates copolymer (Akzo Nobel). The comparative hair styling compositions comprise well-known, synthetic curl styling polymers.

The composition of the compositions used in this example are shown in Table 13.

Table 13. Composition of the tested hair styling compositions

| Sample | Ingredients | | |
|---|---|---|---|
| | Water | Polymers (wt%) | Euxyl PE9010 (wt%) |
| Gosulin Hold 2% | Qs | 2 | 1 |
| Gosulin Style 5% | qs | 5 | 1 |
| PVP K30 5% | Qs | 5 | 1 |
| PU-14 and AMP-acrylates copolymer 5% | qs | 5 | 1 |
| Blank | qs | 0 | 1 |
| Untreated | 0 | 0 | 0 |

Hair pretreatment. The different hair styling compositions were tested on Caucasian, untreated hair swatches (10 cm long and 1 cm wide). Prior to the experiment, the hair swatches were washed with a neutral shampoo, rinsed, and treated with a neutral hair conditioner and dried. The conditioner was used for hair detangling and combing before performing the tests. The composition of the neutral shampoo and the hair conditioner are shown above, in Table 2 and 3, respectively.

The different compositions (5 g) were applied to the dried dry hair swatches. The hair swatches were left to dry at ambient temperature for 4 hours. After this, the hair swatches were pinned on a vertical board and the height of the swatch was measured. The measurement was repeated after 1 h, 4 h, 8 h, 24 h, 36 h, and 72 h.

The height of the hair swatch was measured from the base, where the hair was attached with a tape, to the elevated top of the hair swatch.

The results are compared to a blank composition (without styling polymer).

The stiffness was evaluated according to equation (eq 2).

$$Stiffness = 100\% - \frac{L_0 - L_x}{L_0} * 100\% \qquad (eq\ 2)$$

With:

$L_0$ - initial length of the straight hair swatch
Lx - height of the hair swatch after x h

The stiffness (in %) of the blank composition was 28% for up to 72h. For the Gosulin Style and Gosulin Hold compositions according to the present invention, the height of the hair swatch did not change over the 72h tested: the stiffness remained at 100% at all evaluation times. Similarly, the stiffness for the synthetic styling polymers remained at 100% at all evaluation times.
The Gosulin Hold and Gosulin Style compositions according to the present invention provide the same stiffness as synthetic styling polymers.

***Example 4 - Composition of hair styling compositions according to the present invention.***

Different hair styling compositions comprising different amounts of inulin, particularly branched inulin with DP between 25 and 30, pullulan and cellulose gum (or carboxymethylcellulose) were prepared. The hair styling natural polymers present in these compositions are shown in Table 15 (in wt%). All these combinations have a good styling effect, and have remarkable moisture retaining/moistening properties as well.

Table 15. Concentration of natural polymers present in hair styling compositions according to the present invention.

| Polymer | A (Gosulin Hold 2%) | B | C | D | E (Gosulin Style 5%) | F (Gosulin Style 10%) |
|---|---|---|---|---|---|---|
| Cellulose gum | 1.8 | 0 | 0 | 0 | 0 | 0 |
| Branched Inulin | 0.2 | 5 | 10 | 3.5 | 3.3 | 6.6 |
| pullulan | 0 | 0 | 0 | 1.5 | 1.7 | 3.4 |
| Water | qs | qs | qs | qs | qs | qs |
| Euxyl PE9010 | 1 | 1 | 1 | 1 | 1 | 1 |

Further particular statements (features) and embodiments according to the present disclosure are set herein below.
Statement 1. A hair styling composition, particularly a leave-on hair styling composition, comprising

- a fructooligosaccharide, pullulan, or a combination of a fructooligosaccharide and pullulan,
- optionally a hydroxyalkyl or carboxyalkyl derivative of cellulose, and
- a cosmetically acceptable carrier,

wherein at least one of the fructooligosaccharide, the pullulan, and, optionally, the hydroxyalkyl or carboxyalkyl derivative of cellulose is present in a concentration of at least 1.5 wt%, with wt% based on the total weight of the composition, and
wherein the fructooligosaccharide and/or the pullulan, and, optionally, the hydroxyalkyl or carboxyalkyl derivative of cellulose are present in a combined concentration that is effective as a hair styling or hair fixative agent to style or fix hair into a desired configuration.

Statement 2. The hair styling composition according to statement 1, wherein:

- the fructooligosaccharide and/or the pullulan are each present in a concentration ranging from 0 to 25 wt%, particularly from 0 to 15 wt%,

- the fructooligosaccharide and/or pullulan are present in a combined concentration ranging from 4 to 25 wt%, particularly ranging from 5 to 15 wt%, with wt% based on the total weight of the composition; and
- optionally, the hydroxyalkyl or carboxyalkyl derivative of cellulose is present in a concentration ranging from 1.5 to 3.0 wt%, with wt% based on the total weight of the composition.

Statement 3. The hair styling composition according to statement 1, wherein:

- the composition comprises the hydroxyalkyl or carboxyalkyl derivative of cellulose in a concentration ranging from 1.5 to 3.0 wt%, with wt% based on the total weight of the composition;
- the fructooligosaccharide and/or the pullulan are each present in a concentration ranging from 0 to 5 wt%, particularly ranging from 0 to 4 wt%, and
- the fructooligosaccharide and/or pullulan are present in a combined concentration of less than 5 wt%, particularly less than 4 wt%.

Statement 4. The hair styling composition according to anyone of statements 1 to 3 wherein the fructooligosaccharide is a linear or branched inulin, particularly wherein the inulin is a branched inulin with a degree of polymerization of at least 20, more particularly wherein the branched inulin has a degree of polymerization ranging from 25 to 40, even more particularly wherein the branched inulin has a degree of polymerization ranging from 25 to 35 or from 25 to 30.

Statement 5. The hair styling composition according to claim any one of statements 1 to 4, wherein the hydroxyalkyl or carboxyalkyl derivative of cellulose is carboxymethyl cellulose.

Statement 6. The hair styling composition according to any one of statements 1 to 5,

wherein the cosmetically acceptable carrier is chosen from water, organic solvents and mixtures thereof; and/or

wherein the composition is essentially free of synthetic hair styling or synthetic hair fixative agents and/or of silicones; and/or

wherein the composition further comprises one or more of a fragrance, a colorant, a surfactant, a thickening agent, a vitamin or provitamin, a wax, butter or an oil, an antioxidant, a moisturizer, a chelating agent or a preservative; and/or

wherein the composition is in the form of a spray, a gel, a foam or mousse, a cream, an emulsion or lotion, a liquid, a wax, or a paste.

Statement 7. A method for styling hair, comprising applying the hair styling composition of any one of statements 1 to 6 to the hair and styling the hair with a styling device or by hand.

Statement 8. Use of a fructooligosaccharide, in particular a branched inulin, and/or pullulan, and optionally a hydroxyalkyl or carboxyalkyl derivative of cellulose, for simultaneously moistening and styling hair.

Statement 9. Use according to statement 8 in curl styling applications for providing soft curls, as evaluated by sensory analysis (touch), wherein a fructooligosaccharide, in particular a branched inulin, and/or pullulan is applied to the hair.

Statement 10. Use according to statement 8 in curl styling applications for providing hard curls, as evaluated by sensory analysis (touch), wherein a combination of a fructooligosaccharide, in particular a branched inulin, and/or pullulan, and a hydroxyalkyl or carboxyalkyl derivative of cellulose, in particular carboxymethyl cellulose, is applied to the hair.

**Claims**

1. A hair styling composition, particularly a leave-on hair styling composition, comprising

- a branched fructooligosaccharide having a degree of polymerization between 3 and 100,
- a cosmetically acceptable carrier, and
- optionally, pullulan, a hydroxyalkyl or carboxyalkyl derivative of cellulose, or a combination of pullulan and a hydroxyalkyl or carboxyalkyl derivative of cellulose,

**characterized in that**

the branched fructooligosaccharide is present in a concentration ranging from 0.1 to 25 wt%, particularly from 0.1 to 15 wt%; and

at least one of the branched fructooligosaccharide, the pullulan, if present, and the hydroxyalkyl or carboxyalkyl derivative of cellulose, if present, is present in a concentration of at least 1.5 wt%, with wt% based on the total weight of the composition.

2. The hair styling composition according claim 1, wherein:

- the pullulan is present in a concentration ranging from 0 to 25 wt%, particularly from 0 to 15 wt%,
- the branched fructooligosaccharide and pullulan are present in a combined concentration ranging from 4 to 25 wt%, particularly ranging from 5 to 15 wt%, with wt% based on the total weight of the composition; and
- optionally, the hydroxyalkyl or carboxyalkyl derivative of cellulose is present in a concentration ranging from 1.5 to 3.0 wt%, with wt% based on the total weight of the composition.

3. The hair styling composition according to claim 1, wherein:

- the composition comprises the hydroxyalkyl or carboxyalkyl derivative of cellulose in a concentration ranging from 1.5 to 3.0 wt%, with wt% based on the total weight of the composition;
- the pullulan is present in a concentration ranging from 0 to 5 wt%, particularly ranging from 0 to 4 wt%, and
- the branched fructooligosaccharide and pullulan are present in a combined concentration of less than 5 wt%, particularly less than 4 wt%.

4. The hair styling composition according to anyone of claims 1 to 3 wherein the branched fructooligosaccharide is a branched inulin.

5. The hair styling composition according to claim 4, wherein the branched inulin has a degree of polymerization of at least 20.

6. The hair styling composition according to claim 5, wherein the branched inulin has a degree of polymerization ranging from 25 to 40, more particularly wherein the branched inulin has a degree of polymerization ranging from 25 to 35 or from 25 to 30.

7. The hair styling composition according to claim any one of claims 1 to 6, wherein the hydroxyalkyl or carboxyalkyl derivative of cellulose is carboxymethyl cellulose.

8. The hair styling composition according to any one of claims 1 to 7, wherein the cosmetically acceptable carrier is chosen from water, organic solvents and mixtures thereof.

9. The hair styling composition according to any one of claims 1 to 8, wherein the composition is essentially free of synthetic hair styling or synthetic hair fixative agents and/or of silicones.

10. The hair styling composition according to any one of claims 1 to 9, wherein the composition further comprises one or more of a fragrance, a colorant, a surfactant, a thickening agent, a vitamin or provitamin, a wax, butter or an oil, an antioxidant, a moisturizer, a chelating agent or a preservative.

11. The hair styling composition according to any one of claims 1 to 10, wherein the composition is in the form of a spray, a gel, a foam or mousse, a cream, an emulsion or lotion, a liquid, a wax, or a paste.

12. A method for styling hair, comprising applying the hair styling composition of any one of claims 1 to 11 to the hair and styling the hair with a styling device or by hand.

13. Use of a branched fructooligosaccharide, in particular a branched inulin, and, optionally, pullulan and/or a hydroxyalkyl or carboxyalkyl derivative of cellulose, for simultaneously moistening and styling hair.

14. Use according to claim 13 in curl styling applications for providing soft curls, as evaluated by sensory analysis (touch), wherein a branched fructooligosaccharide, in particular a branched inulin, and, optionally, pullulan is applied to the hair.

15. Use according to claim 13 in curl styling applications for providing hard curls, as evaluated by sensory analysis (touch), wherein a combination of a branched fructooligosaccharide, in particular a branched inulin, a carboxyalkyl derivative of cellulose, in particular carboxymethyl cellulose, and, optionally, pullulan, is applied to the hair.

Europäisches
Patentamt

European
Patent Office

Office européen
des brevets

**EUROPEAN SEARCH REPORT**

Application Number

EP 24 15 9262

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE GNPD [Online] MINTEL; 1 December 2022 (2022-12-01), anonymous: "Honey Styling Gel", XP093080568, Database accession no. 10398552 | 1-15 | INV. A61K8/60 A61Q5/06 |
| Y | * abstract * | 1-15 | |
| X,P | DATABASE GNPD [Online] MINTEL; 12 June 2023 (2023-06-12), anonymous: "Volumising Hair Spray", XP093181593, Database accession no. 10869594 * abstract * | 1-15 | |
| X | JP 2022 079579 A (NIPPON FINE CHEMICAL CO) 26 May 2022 (2022-05-26) * paragraph [0050]; examples 12,50 * | 1,2,4,5, 8-11 | |
| X | JP 2018 188403 A (NIPPON FINE CHEMICAL CO; FUJI NIHON SEITO KK) 29 November 2018 (2018-11-29) | 1-15 | TECHNICAL FIELDS SEARCHED (IPC) A61K |
| Y | * paragraphs [0016] - [0020]; examples 40,42,43,... * | 1-15 | A61Q |
| X | US 2019/328637 A1 (PARIKH DHARA [US] ET AL) 31 October 2019 (2019-10-31) | 1-15 | |
| Y | * paragraph [0032]; example 1 * | 1-15 | |
| X | US 9 918 923 B1 (NAIBERK EMMA [US] ET AL) 20 March 2018 (2018-03-20) | 1-15 | |
| Y | * table 1 * | 1-15 | |

-/--

The present search report has been drawn up for all claims

| Place of search | Date of completion of the search | Examiner |
|---|---|---|
| Munich | 16 July 2024 | Skulj, Primoz |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

Europäisches
Patentamt

European
Patent Office

Office européen
des brevets

## EUROPEAN SEARCH REPORT

**Application Number**

EP 24 15 9262

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | Lebrilla C B ET AL: "Oligosaccharides and Polysaccharides - Essentials of Glycobiology - NCBI Bookshelf", , 31 December 2022 (2022-12-31), XP093085380, Retrieved from the Internet: URL:https://www.ncbi.nlm.nih.gov/books/NBK579972/ [retrieved on 2023-09-25] ----- | | |

TECHNICAL FIELDS
SEARCHED      (IPC)

The present search report has been drawn up for all claims

| Place of search | Date of completion of the search | Examiner |
|---|---|---|
| Munich | 16 July 2024 | Skulj, Primoz |

EPO FORM 1503 03.82 (P04C01)

## EP 4 427 731 A1

### ANNEX TO THE EUROPEAN SEARCH REPORT
### ON EUROPEAN PATENT APPLICATION NO.

EP 24 15 9262

This annex lists the patent family members relating to the patent documents cited in the above-mentioned European search report.
The members are as contained in the European Patent Office EDP file on
The European Patent Office is in no way liable for these particulars which are merely given for the purpose of information.

16-07-2024

| Patent document cited in search report | | Publication date | Patent family member(s) | | Publication date |
|---|---|---|---|---|---|
| JP 2022079579 | A | 26-05-2022 | JP | 7141628 B2 | 26-09-2022 |
| | | | JP | 2018188403 A | 29-11-2018 |
| | | | JP | 2022079579 A | 26-05-2022 |
| | | | JP | 2024019506 A | 09-02-2024 |
| JP 2018188403 | A | 29-11-2018 | JP | 7141628 B2 | 26-09-2022 |
| | | | JP | 2018188403 A | 29-11-2018 |
| | | | JP | 2022079579 A | 26-05-2022 |
| | | | JP | 2024019506 A | 09-02-2024 |
| US 2019328637 | A1 | 31-10-2019 | US | 2019328637 A1 | 31-10-2019 |
| | | | WO | 2019212710 A1 | 07-11-2019 |
| US 9918923 | B1 | 20-03-2018 | NONE | | |

EPO FORM P0459

**EP 4 427 731 A1**

**REFERENCES CITED IN THE DESCRIPTION**

**Non-patent literature cited in the description**

- **MANCILLA-MARGALLI ; LOPEZ.** *J. Agric. Food Chem,* 2006, vol. 54, 7832-7839